# EUROPEAN PATENT APPLICATION

(11) **EP 2 186 785 A2**
(43) Date of publication of application: **19.05.2010**
(21) Application number: 10151828.0
(22) Date of filing: 27.01.2010
(51) Int. Cl.: C07C 7/13, C07C 11/02, C07C 303/06, C07C 309/20

(54) **Process for the separation of olefins from paraffins**

(71) Applicant: Shell Internationale Research Maatschappij B.V., 2596 HR The Hague (NL)
(72) Inventor: Dirkzwager, Hendrik, 1031 HW, Amsterdam (NL); Moene, Robert, 1031 HW, Amsterdam (NL)

(57) **Abstract**

An adsorptive separation process for the separation of olefins having in the range of from 20 to 28 carbon atoms from a feed stream comprising one or more olefins and paraffins, comprising contacting the feed stream with a bed of adsorbent under conditions which cause the selective retention of the olefins having in the range of from 20 to 28 carbon atoms on the adsorbent and recovering the retained olefins having in the range of from 20 to 28 carbon atoms from the adsorbent by contacting the adsorbent with a desorbent.

## Description

### Field of the Invention

The present invention relates to a process for the separation of olefins, in particular olefins having in the range of from 20 to 28 carbon atoms, from paraffins.

### Background of the Invention

Heavy internal olefins (e.g. C15-C30) are needed for the production of Internal Olefin Sulphonates (IOS) and/or their derivatives (according to EP 351928 A1).

Branched and linear heavy internal olefins are useful surfactant-precursors to be applied in enhanced oil recovery (EOR).

Furthermore, the internal olefin derived alcohols and/or alkoxylates may serve this purpose after conversion to anionic surfactants by sulphation.

Appropriate internal higher olefin feeds can be obtained from the Shell Higher Olefin Process (SHOP) process and 1-olefin production processes as employed by Chevron-Phillips Chemical Company or INEOS (BP-AMOCO). However, as it is expected that large amounts of surfactants and hence, heavy internal olefins (e.g. C15-C30), are required for EOR, it is desirable if such olefins can also be obtained from other sources.

The dehydrogenation of paraffins leads to a mixture of olefins which mostly contain internal double bonds. As described in Encyclopedia of Chemical Professing and Design, 1982, Vol. 15, pp 266-284, a commercial process for the dehydrogenation of paraffins according is UOP's "PACOL" process. This process gives rise to the production of paraffin-diluted internal olefins (generally < 20 percent by weight (wt. %) olefin content), as at higher conversions per pass dienes and aromatics are formed as by-products to a large extent and moreover, coking phenomena are encountered in the "PACOL" dehydrogenation unit.

Furthermore, it is necessary to separate olefins from paraffins in the resulting product mixture. However, separation may be difficult or impossible by distillation due to the similar volatilities of components in the product mixture and adsorptive separation is often used in order to separate olefins from paraffins.

However, hitherto said process has been directed to preparing lower carbon number olefins for use, in particular, as feedstock for detergent alcohol manufacture.

For example, US 2008/0051619 A1 describes a process for the separation of detergent-range olefinic hydrocarbons from a feed stream comprising one or more olefinic hydrocarbons and other hydrocarbon species using a specific type of hydrocarbon as desorbent.

Hence, a cheap and straightforward technique is required for preparing heavy olefins, and in particular heavy internal olefins, for use as surfactant precursors.

### Summary of the Invention

According to the present invention there is provided an adsorptive separation process for the separation of olefins having in the range of from 20 to 28 carbon atoms from a feed stream comprising one or more olefins and paraffins, comprising contacting the feed stream with a bed of adsorbent under conditions which cause the selective retention of the olefins having in the range of from 20 to 28 carbon atoms on the adsorbent and recovering the retained olefins having in the range of from 20 to 28 carbon atoms from the adsorbent by contacting the adsorbent with a desorbent.

### Detailed Description of the Invention

Various processes produce mixtures of paraffins and olefins. The feed stream for separation in the process of the present invention may be any stream comprising one or more olefins and paraffins, wherein said olefins have in the range of from 20 to 28 carbon atoms.

In a preferred embodiment, said feed stream is a Fischer-Tropsch derived feed stream, that is to say, a product stream from Fischer-Tropsch synthesis (e.g. a gas-to-liquids (GTL), a biomass-to-liquids (BTL) or a coal-to-liquids (CTL) product).

The Fischer-Tropsch process is well known to those skilled in the art and can be used for the conversion of synthesis gas (from hydrocarbonaceous feed stocks) into liquid and/or solid hydrocarbons. Generally, the feed stock (e.g. natural gas, associated gas and/or coal-bed methane, heavy and/or residual oil fractions, coal, biomass) is converted in a first step into a mixture of hydrogen and carbon monoxide (this mixture is often referred to as "synthesis gas" or "syngas").

The synthesis gas is then fed into a reactor where it is converted in one or more steps over a suitable catalyst at elevated temperature and pressure into mainly paraffinic compounds and water. The obtained paraffinic compounds range from methane to high molecular weight molecules. The obtained high molecular weight molecules can comprise up to 200 carbon atoms, or, under particular circumstances, even more carbon atoms.

Numerous types of reactor systems have been developed for carrying out the Fischer-Tropsch reaction. For example, Fischer-Tropsch reactor systems include fixed bed reactors, especially multi-tubular fixed bed reactors, fluidised bed reactors, such as entrained fluidised bed reactors and fixed fluidised bed reactors, and slurry bed reactors such as three-phase slurry bubble columns and ebulated bed reactors.

Catalysts used in the Fischer-Tropsch synthesis often comprise a carrier based support material and one or more metals from Group VIII (IUPAC 8-10) of the Periodic Table of Elements, especially from the cobalt or iron groups, optionally in combination with one or more metal oxides and/or metals as promoters selected from zirconium, titanium, chromium, vanadium and manganese, especially manganese. Such catalysts are known in the art and have been described for example, in the specifications of WO 97/00231 A and US 4595703 A.

The synthesis gas can be provided by any suitable means, process or arrangement. This includes partial oxidation and/or reforming of a hydrocarbonaceous feedstock as is known in the art. To adjust the H₂/CO ratio in the synthesis gas, carbon dioxide and/or steam may be introduced into the partial oxidation process.
The H₂/CO ratio of the synthesis gas is suitably between 1.5 and 2.3, preferably between 1.6 and 2.0.

The synthesis gas comprising predominantly hydrogen, carbon monoxide and optionally nitrogen, carbon dioxide and/or steam is contacted with a suitable catalyst in the catalytic conversion stage, in which the hydrocarbons are formed. Suitably at least 70 v/v% of the synthesis gas is contacted with the catalyst, preferably at least 80%, more preferably at least 90 %, still more preferably all the synthesis gas.

A steady state catalytic hydrocarbon synthesis process may be performed under conventional synthesis conditions known in the art. Typically, the catalytic conversion may be effected at a temperature in the range of from 100 to 600 °C, preferably from 150 to 350 °C, more preferably from 175 to 275 °C, most preferably 200 to 260 °C. Typical total pressures for the catalytic conversion process are in the range of from 5 to 150 bar absolute, more preferably from 5 to 80 bar absolute. In the catalytic conversion process mainly C₅+ hydrocarbons are formed.

A suitable regime for carrying out the Fischer-Tropsch process with a catalyst comprising particles with a size of least 1 mm is a fixed bed regime, especially a trickle flow regime. A very suitable reactor is a multi-tubular fixed bed reactor.

Products of the Fischer-Tropsch synthesis may range from methane to heavy paraffin waxes. Preferably, the production of methane is minimised and a substantial portion of the hydrocarbons produced have a carbon chain length of a least 5 carbon atoms. Preferably, the amount of C₅+ hydrocarbons is at least 60 wt. % of the total product, more preferably, at least 70 wt. %, even more preferably, at least 80 wt. %, most preferably at least 85 wt. %.

The hydrocarbons produced in the Fischer-Tropsch process are suitably C3-200 hydrocarbons, more suitably C4-150 hydrocarbons, especially C5-100 hydrocarbons, or mixtures thereof. These hydrocarbons or mixtures thereof are liquid or solid at temperatures between 5 and 30 °C (1 bar), especially at about 20 °C (1 bar), and usually are paraffinic in nature, while up to 30 wt. %, preferably up to 15 wt. %, of either olefins or oxygenated compounds may be present.

Depending on the catalyst and the process conditions used in a Fischer-Tropsch reaction, various proportions of normally gaseous hydrocarbons, normally liquid hydrocarbons and optionally normally solid hydrocarbons are obtained. It is often preferred to obtain a large fraction of normally solid hydrocarbons. These solid hydrocarbons may be obtained up to 90 wt. % based on total hydrocarbons, usually between 50 and 80 wt. %.

A part may boil above the boiling point range of the so-called middle distillates. The term "middle distillates", as used herein, is a reference to hydrocarbon mixtures of which the boiling point range corresponds substantially to that of kerosene and gasoil fractions obtained in a conventional atmospheric distillation of crude mineral oil. The boiling point range of middle distillates generally lies within the range of from 150 to 360 °C.

The higher boiling range paraffinic hydrocarbons, if present, may be isolated and subjected to a catalytic hydrocracking step, which is known per se in the art, to yield the desired middle distillates. The catalytic hydrocracking is carried out by contacting the paraffinic hydrocarbons at elevated temperature and pressure and in the presence of hydrogen with a catalyst containing one or more metals having hydrogenation activity, and supported on a support comprising an acidic function. Suitable hydrocracking catalysts include catalysts comprising metals selected from Groups VIB (IUPAC 6) and VIII (IUPAC 8-10) of the Periodic Table of Elements. Preferably, the hydrocracking catalysts contain one or more noble metals from Group VIII (IUPAC 8-10) of the Periodic Table of Elements. Preferred noble metals are platinum, palladium, rhodium, ruthenium, iridium and osmium. Most preferred catalysts for use in the hydrocracking stage are those comprising platinum.

The amount of catalytically active noble metal present in the hydrocracking catalyst may vary within wide limits and is typically in the range of from 0.05 to 5 parts by weight per 100 parts by weight of the support material. The amount of non-noble metal present is preferably 5-60 %, more preferably 10-50 %.

Suitable conditions for the catalytic hydrocracking are known in the art. Typically, the hydrocracking is effected at a temperature in the range of from 175 to 400 °C. Typical hydrogen partial pressures applied in the hydrocracking process are in the range of from 10 to 250 bar.

The product of the hydrocarbon synthesis and consequent hydrocracking suitably comprises mainly normally liquid hydrocarbons, beside water and normally gaseous hydrocarbons. By selecting the catalyst and the process conditions in such a way that especially normally liquid hydrocarbons are obtained, the product obtained ("syncrude") may be transported in the liquid form or be mixed with any stream of crude oil without creating any problems as to solidification and or crystallization of the mixture. It is observed in this respect that the production of heavy hydrocarbons, comprising large amounts of solid wax, are less suitable for mixing with crude oil while transport in the liquid form has to be done at elevated temperatures, which is less desired.

Thus, the feed stream used in the process of the present invention may have undergone the steps of hydroprocessing, hydrogenation, hydroisomerisation and/or hydrocracking. Said feed stream may also be a fuel, preferably gasoil, a waxy raffinate or a base oil.

Consequently, in a preferred embodiment of the present invention, said feed stream may be any product stream from a Fischer-Tropsch process which comprises olefins and paraffins having in the range of from 20 to 28 carbon atoms. That is to say, the feed stream may be the direct product stream from the Fischer-Tropsch paraffin synthesis unit (HPS) or it may be a product stream derived therefrom after subsequent processing such as hydrogenation, for example, as described below.

In the Fischer-Tropsch process, light ends (C5-) and a Light Detergent Feedstock (LDF, C10-C13) are typically removed by distillation from the product stream from the HPS unit. The Light Detergent Feedstock (LDF, C10-C13) stream is subsequently subjected to hydrogenation. The remainder of the HPS effluent is then typically sent to a hydrocracking unit (HPC). The effluent from the HPC unit is then typically distilled in a syncrude distillation unit (SCD) to produce products ranging from LPG to Gasoil. The heavy ends from the SCD unit are sent to vacuum distillation unit (HVU) where fractions to be used for base oils are removed before the heavies are recycled back to the HPC unit. The base oil fractions are then sent to a catalytic dewaxing unit (CDW) before being distilled into final base oil product fractions.

Hence, additional sources of paraffins having in the range of from 20 to 28 carbon atoms within the Fischer-Tropsch process not only include product streams directly from the HPS unit, but may also include product streams from the hydrocracking unit (HPC) and certain base oil product fractions.

Said Fischer-Tropsch derived feed stream may be used directly in order to separate olefins therein from paraffins.

In a preferred embodiment, the feed stream for use in the process of the present invention will have undergone a dehydrogenation step prior to the process of the present invention in order to dehydrogenate at least a portion of the paraffins therein to form olefins, that is to say, to increase the amount of olefins therein prior to undergoing separation by the process of the present invention. Hence, preferably the feed stream for use in the process of the present invention is derived from a paraffin dehydrogenation process.

In a particularly preferred embodiment, the feed stream is a Fischer-Tropsch derived feed stream which has subsequently undergone a paraffin dehydrogenation process. That is to say, the feed stream may be considered as not only being a Fischer-Tropsch derived feed stream, but also as being derived from a paraffin dehydrogenation process.

The feed stream for use in the process of the present invention may comprise mixtures of linear and/or branched olefins and paraffins.

Oil reservoirs have variable characteristics such as crude oil type, salinity and temperature. Consequently, surfactants such as internal olefin sulphonates for use in enhanced oil recovery applications are tailored dependent upon the specific characteristics of the oil reservoir being treated.

Accordingly, in one preferred embodiment of the present invention the feed stream comprises olefins and paraffins having in the range of from 20 to 24 carbon atoms, whilst in another preferred embodiment of the present invention the feed stream comprises olefins and paraffins having in the range of from 24 to 28 carbon atoms.

In embodiments of the present invention wherein the feed stream is derived from a Fischer-Tropsch process and has optionally undergone a subsequent paraffin dehydrogenation process, said feed stream for use in the process of the present invention may comprise different levels of olefins, in particular branched olefins, depending upon the exact source of the feed stream from the Fischer-Tropsch process.

For example, in one embodiment of the present invention, when the Fischer-Tropsch derived feed stream is from the HPS unit, it is preferred that olefins having in the range of from 20 to 28 carbon atoms, more preferably in the range of from 20 to 24 carbon atoms or 24 to 28 carbon atoms, are present in the feed stream in an amount of in the range of from 5 to 15 wt. %, with respect to the total weight of said Fischer-Tropsch derived feed stream having said carbon number range and that said feed stream comprises at least 5 wt. % of branched olefins having in the range of from 20 to 28 carbon atoms, more preferably in the range of from 20 to 24 carbon atoms or 24 to 28 carbon atoms, with respect to the total weight of olefins in said carbon number range in the Fischer-Tropsch derived feed stream.

In another embodiment of the present invention, when the Fischer-Tropsch derived feed stream is from the HPC unit, it is preferred that olefins having in the range of from 20 to 28 carbon atoms, more preferably in the range of from 20 to 24 carbon atoms or 24 to 28 carbon atoms, are present in the feed stream in an amount of in the range of from 5 to 15 wt. %, with respect to the total weight of said Fischer-Tropsch derived feed stream having said carbon number range and that said feed stream comprises at least 50 wt. % of branched olefins having in the range of from 20 to 28 carbon atoms, more preferably in the range of from 20 to 24 carbon atoms or 24 to 28 carbon atoms, with respect to the total weight of olefins in said carbon number range in the Fischer-Tropsch derived feed stream.

In a further embodiment of the present invention, when the Fischer-Tropsch derived feed stream is a base oil product fraction, it is preferred that olefins having in the range of from 20 to 28 carbon atoms, more preferably in the range of from 20 to 24 carbon atoms or 24 to 28 carbon atoms, are present in the feed stream in an amount in the range of from 5 to 15 wt. %, with respect to the total weight of said Fischer-Tropsch derived feed stream having said carbon number range and that said feed stream comprises at least 90 wt. % of branched olefins having in the range of from 20 to 28 carbon atoms, more preferably in the range of from 20 to 24 carbon atoms or 24 to 28 carbon atoms, with respect to the total weight of olefins in said carbon number range in the Fischer-Tropsch derived feed stream.

In the event that the feed stream for use in the process of the present invention has been derived from a paraffin dehydrogenation process, then said dehydrogenation process is not limited and may be any paraffin dehydrogenation process known in the art.

The or each dehydrogenation reaction zone may be operated in a continuous-type or batch-type manner.

The dehydrogenation catalyst used in the or each dehydrogenation zone of the process of the present invention may be in a fixed bed, a moving catalyst bed or a fluidised bed.

The choice of a dehydrogenation catalyst is not limited and suitable procedures for preparing catalysts and performing dehydrogenation include those described in US 3274287 A, US 3315007 A, US 3315008 A, US 3745112 A, US 4048246 A, US 4079097 A, US 4080394 A, US 4304950 A, US 4430517 A, US 4458098 A, US 4506032 A, US 4595673 A, US 4677237 A, US 4716143 A, US 4762960 A, US 4786625 A, US 4827072 A, US 5012021 A, US 6187981 B1, US 6756515 B1, US 2003/0202934 A1, CA 928330 A, WO 02/41990 A1, WO 2004/072004 and WO 2005/037753.

Preferred dehydrogenation catalyst compositions comprise at least one platinum group (Group VIII (IUPAC 8-10) of the Periodic Table of Elements) metal, a promoter metal, a modifier metal, and a porous carrier material.

The platinum group (Group VIII (IUPAC 8-10) of the Periodic Table of Elements) metals which may be used include platinum, palladium, rhodium, iridium, ruthenium, and osmium.

Promoter metals may be conveniently selected from the group consisting of tin, germanium, rhenium, gallium, bismuth, lead, indium, cerium, zinc, and mixtures thereof.

Modifier metals may conveniently be selected from the group consisting of alkali metals, alkaline earth metals and mixtures thereof.

The alkali and alkaline earth metals which can be used as modifier metals in the dehydrogenation catalyst composition include lithium, sodium, potassium, caesium, rubidium, beryllium, magnesium, calcium, strontium, and barium. Preferred modifier metals are lithium, potassium, sodium, and caesium with lithium and potassium being especially preferred. The modifier metal may exist in the final catalyst composition in an oxidation state above that of the platinum group (Group VIII (IUPAC 8-10) of the Periodic Table of Elements) metal. The modifier metal may be present as an oxide. In some embodiments, the modifier metal may be combined with the porous carrier material. In certain embodiments, the modifier metal may be combined with other dehydrogenation catalyst components.

The porous carrier material used in the dehydrogenation catalyst composition may include a porous, absorptive support with high surface area in the range of from 25 m²/g to 500 m²/g. The porous carrier material may have a melting point greater than the conditions utilised in the dehydrogenation zone.

Examples of carrier materials include, but are not limited to, activated carbon, coke, charcoal, silica, silica gel, silicon carbide, synthetically prepared and/or naturally occurring clays and silicates, refractory inorganic oxides, crystalline zeolitic aluminosilicates, naturally occurring or synthetically prepared mordenite and/or faujasite, spinels or combinations of thereof.

In some embodiments, clays and silicates may or may not be acid treated (e.g., attapulgite, china clay, diatomaceous earth, fuller's earth, kaolin, kieselguhr, ceramics, porcelain, crushed firebrick, bauxite).

Examples of refractory inorganic oxides include alumina, titanium dioxide, zirconium dioxide, chromium oxide, beryllium oxide, vanadium oxide, cerium oxide, hafnium oxide, zinc oxide, magnesia, boria, thoria, silica-alumina, silica-magnesia, chromia-alumina, alumina-boria, and silica-zirconia.

Zeolitic aluminosilicates may be, in some embodiments, in the hydrogen form. In other embodiments, zeolitic aluminosilicates may be in a form that may be exchanged with metal cations. Examples of spinels include, but are not limited to, MgAl₂O₄, FeAl₂O₄, ZnAl₂O₄, CaAl₂O₄, and other like compounds having the formula MO-Al₂O₃ in which M is a metal having a valence of 2.

Preferred porous carrier materials are refractory inorganic oxides selected from alpha alumina, theta alumina, cordierite, zirconia, titania, and mixtures thereof. Particularly preferred porous carrier materials are alpha alumina and cordierite.

Although the concentration of each metal component can vary substantially, it is preferable that the platinum group (Group VIII (IUPAC 8-10) of the Periodic Table of Elements) metal be present in a concentration in the range of from 0.01 to 5 wt. % on an elemental basis of the total weight of the catalyst composition, more preferably in the range of from 0.1 to 1.0 wt. % and most preferably in the range of from 0.05 to 1.0 wt. % on an elemental basis of the total weight of the catalyst composition.

The promoter metal is preferably present in an amount in the range of from 0.01 to 5 wt. %, more preferably in the range of from 0.05 to 5 wt. % of the total weight of the catalyst composition

The modifier metal is preferably present in an amount in the range of from 0.1 to 5 wt. % and more preferably in the range of from 2 to 4 wt. % of the total weight of the catalyst composition.

The atomic ratio of the platinum group (Group VIII (IUPAC 8-10) of the Periodic Table of Elements) metal to modifier metal may vary in the range of from 0.05 to 5. In particular, when the modifier metal is tin, the atomic ratio is typically in the range of from 0.1:1 to 5:1 and preferably in the range of from 0.5:1 to 3:1. When the modifier metal is germanium the ratio is typically in the range of from 0.25:1 to 5:1 and when the promoter metal is rhenium, the ratio is typically in the range of from 0.05:1 to 2.75:1.

In some embodiments, the dehydrogenation catalyst composition may additionally comprise a halogen component. The halogen component may include, but is not limited to, fluorine, chlorine, bromine, iodine or mixtures thereof. The halogen component may be present in a combined state with the porous carrier material. In certain embodiments, a halogen component may be dispersed throughout the catalyst composition. A halogen component may range from 0.2 to 15 wt. %, calculated on an elemental basis, of the final catalyst composition. In certain embodiments, the dehydrogenation catalyst composition may contain in the range of from 1 to 3 wt. % chlorine.

In certain embodiments, the catalyst composition may include at least 0.2 wt. %, calculated on an elemental basis, of a halogen component. The presence of a halogen component in the catalyst composition may improve the dehydrogenation activity of the catalyst. In some embodiments, the presence of an active halogen component may suppress carbon formation on the catalyst composition during the dehydrogenation process. An additional advantage of the catalyst composition may be that undesirable isomerisation or cracking side reactions may be inhibited. In certain embodiments, the halogen content may increase the acidity of the catalyst composition. Consequently, the acidity may be lowered by steaming the dehydrogenation catalyst composition to remove excess halogen therefrom.

In some embodiments, the dehydrogenation catalyst composition may include a sulfur component in an amount ranging from 0.01 to 10 wt. %, calculated on an elemental basis, of the final catalyst composition.

The dehydrogenation catalyst composition, in some embodiments, may also contain other, additional components or mixtures thereof, which act alone or in concert, as catalyst modifiers to further improve catalyst activity, selectivity or stability.

Examples of catalyst modifiers include, but are not limited to, antimony, arsenic, beryllium, bismuth, cadmium, calcium, chromium, cobalt, copper, gallium, gold, indium, iron, lithium, manganese, molybdenum, nickel, rhenium, scandium, silver, tantalum, thallium, titanium, tungsten, uranium, zinc and zirconium.

A description of a preferred dehydrogenation catalyst may be found in US 4506032 A.

The dehydrogenation conditions may be conveniently selected to minimise cracking and formation of diene and aromatic by-products. When contacting the catalyst, the paraffins may be in the liquid phase or in a mixed vapour-liquid phase, but preferably the paraffins are in the vapour phase.

Typical dehydrogenation conditions include temperatures of preferably in the range of from 400 °C (752 °F) to 900 °C (1652 °F) and more preferably in the range of from 400 °C (752 °F) to 525 °C (977 °F), pressures of preferably in the range of from 1 kPa(g) (0.15 psi(g)) to 1013 kPa(g) (147 psi(g)), more preferably in the range of from 1 kPa(g) (0.15 psi(g)) to 345 kPa(g) (50 psi(g))and a preferred LHSV in the range of from 0.1 to 100 hr-1. As used herein, the abbreviation "LHSV" means liquid hourly space velocity, which is defined as the volumetric flow rate of liquid per hour divided by the catalyst volume, where the liquid volume and the catalyst volume are in the same volumetric units.

The olefin-containing dehydrogenated product stream from the paraffin dehydrogenation process is typically a mixture of unreacted linear and branched paraffins, linear and branched olefins, in particular linear and branched monoolefins.

The dehydrogenated product stream may undergo a selective hydrogenation step to eliminate diolefins, as described in US 4523045 A prior to separation and recovery of the monoolefin products from the dehydrogenated product stream. It is also possible for the aromatic by-products and any remaining diolefins in the dehydrogenated product stream to be removed by a separate fixed bed adsorption step.

As hereinbefore described, surfactants for use in enhanced oil recovery applications are tailored depending upon the specific characteristics of the oil reservoir being treated. Consequently, branched monoolefins, linear monoolefins and/or mixtures thereof may be preferred for use in surfactant production as described herein, dependent upon the oil reservoir in which the surfactant is to be used. Similarly, alpha monoolefins, internal olefins and/or mixtures thereof may be preferred for use in surfactant production as described herein, dependent upon the oil reservoir in which the surfactant is to be used.

As described in US 2008/0051619 A, an adsorptive separation process comprises an adsorption step wherein an adsorbent is brought into contact with the olefin-containing feed stream under adsorptive conditions and a desorption step wherein selectively adsorbed olefins are removed from the adsorbent under desorption conditions.

Adsorptive separations can be carried out in the vapour phase and in the liquid phase. However, liquid phase separations as preferred as lower temperatures can be used, thereby minimising olefin polymerisation.

The separation zone in the process of the present invention may comprise swing-bed type operations (for example, as disclosed in US 2920037 A) or continuous counter-current flow bed operations.

In a preferred embodiment, the process of the present invention is a simulated-moving-bed adsorptive separation process.

Typically, simulated-moving-bed processes simulate countercurrent movement of the adsorbent and the feed stream. Such processes are described in Kirk-Othmer Encyclopedia of Chemical Technology (4th Edition, Wiley, New York (1991), Volume 1, pp 583-585 (Adsorption, Liquid Separation section)).

A paper entitled "Olex: A Process for Producing High Purity Olefins" presented by J.A. Johnson, S. Raghuram and P.R. Pujado at the August 1987 Summer national meeting of the American Institute of Chemical Engineers describes a simulated-moving-bed (SMB) countercurrent adsorptive separation process for the separation of light straight-chain olefins from similar paraffins. Detailed descriptions of SMB are also given in US 3510423 A and US 2008/0051619 A1.

As indicated in US 2008/0051619 A, SMB processes often include at least three or four separate steps. Said steps are performed sequentially in separate zones within a mass of adsorbent retained in one or more vertical cylindrical adsorption chambers. The afore-mentioned zones are usually made up of a plurality of adsorbent beds.

The adsorbent beds are contained in one or more vertical vessels which may be collectively referred to as the "adsorbent chamber".

As described in US 2008/0051619 A1, the adsorbent beds are structurally separated from one another by a horizontal liquid collection/distribution grid, each grid being connected to a transfer line defining a transfer point at which process streams such as the feed stream and raffinate and extract streams enter or leave the vertical adsorption vessels.

In the process of the present invention, olefins from the feed stream are selectively adsorbed by the adsorbent and the resulting "raffinate stream" comprises hydrocarbons, in particular paraffins from the feed stream, which are less selectively adsorbed by the adsorbent. The raffinate steam is then removed from the adsorption vessel.

Adsorbents that may be conveniently used in the process of the present invention may be varied depending upon the nature of the olefins to be extracted from the feed stream. Examples of adsorbents that may be used include molecular sieves.

Examples of preferred adsorbents are molecular sieves formed from inorganic oxides such as silica and alumina, that is to say, aluminosilicates. Aluminosilicates include the well known commercially available zeolites such as zeolite Y and zeolite X. Examples of adsorptive separation processes utilising crystalline aluminosilicate adsorbents are disclosed in US 3617504 A, US 3878128 A, US 3929669 A, US 3969223 A, US 4036744 A, US 4048111 A and US 4523045 A.

Molecular sieve zeolites contain exchangeable cationic sites. By ion-exchange using known methods, it is possible for said sites to contain one or more metal cations selected from the metals of Groups I-A, I-B, II-B and II-A (IUPAC 1, 11, 12 and 2) of the Periodic Table of Elements. Such ion exchange typically effects an increase in capacity of the zeolites for the adsorption of olefins. Preferred metal cations for ion exchange include beryllium, lithium, sodium, magnesium, potassium, calcium, rubidium, strontium, caesium, barium, gold, copper, silver, zinc and cadmium.

In one embodiment, it is preferred that said metal cations are selected from Groups I-A, I-B and II-A (IUPAC 1, 11 and 2) of the Periodic Table of Elements. It is particularly preferred that the metal cation is sodium.

However, in another embodiment, silver- and copper-exchanged zeolites, especially silver- and copper-exchanged Type X structured zeolites, are particularly preferred as adsorbents. Such zeolites are known in the art and are described, for example, in US 3718580 A, US 3755153 A and US 3755540 A.

Furthermore, the adsorbent for use in the process of the present invention may be a silicalite, that is to say, a very high silica to alumina ratio molecular sieve which is not a zeolite due to its lack of ion exchange capacity. Such materials are described in US 4061724 A, US 4073865 A and US 4104294 A.

ZSM type zeolites may also be used as the adsorbent in the process of the present invention. Such inorganic oxide molecular sieves are described in US 3702886 A (ZSM-5), US 3832449 A (ZSM-12), US 4016245 A (ZSM-35) and US 4046859 A (ZSM-38).

US 2008/0051619 A1 and US 6225518 B1 describe an example of a preferred adsorbent which is an attrition resistant particle of about 20-40 mesh (U.S.) size formed by extrusion or spray drying an admixture of a binder such as clay or alumina and a type X or type Y zeolite. The type X and type Y zeolites are described in US 2822244 A and US 3130007 A, respectively. Said zeolites may be ion exchanged to replace native sodium with one or more other cations selected from alkali metals and/or alkaline-earth metals. Preferred metals are one or more of lithium, potassium, calcium, strontium and barium. A particularly preferred adsorbent is a sodium form 13X zeolite.

The olefins extracted from the feed stream by the adsorbent may be desorbed therefrom by using a desorbent, that is to say, a material which facilitates desorption of the olefins from the absorbent to form a so-called "extract stream".

Preferred desorbents for use in the process of the present invention are liquids which are able to displace olefins from the adsorbent with reasonable mass flow rates whilst not being so strongly adsorbed themselves as to unduly prevent olefins from displacing the desorbent from the adsorbent in a subsequent adsorption cycle.

Furthermore, preferred desorbents are compatible with the adsorbent and the feed stream. In particular, it is preferred that said desorbents should not reduce or destroy the capacity of the adsorbent or selectivity of the adsorbent for olefins vis-à-vis paraffins in the feed stream. In addition, desorbents should be inert materials, that is to say, said materials should not chemically react with or cause a chemical reaction with components of the feed stream, raffinate stream and extract stream.

Desorbents should also be easily separated from the extract and raffinate components, for example by fractionation.

Desorbents that may be conveniently used in the process of the present invention are compounds having in the range of from 5 to 18 carbons, in particular, hydrocarbons such as paraffins, naphthenic hydrocarbons, olefins and aromatic hydrocarbons having in the range of from 5 to 18 carbons.

The extract stream comprising olefins having in the range of from 20 to 28 carbon atoms and the raffinate stream generally are passed to separation means, typically fractional distillation columns, where at least a portion of desorbent is recovered and an extract product comprising olefins having in the range of from 20 to 28 carbon atoms and a raffinate product are produced.

One operational problem commonly described in the art in relation to the adsorptive separation of olefins is the possible accumulation of certain compounds present in the feed stream, on the active sites of the adsorbent. Such compounds, for example aromatic hydrocarbons and diolefins, act as poisons by possibly binding so tightly to the active sites of the adsorbent that the desorption procedure used for olefin recovery cannot not remove them.

Accordingly, the capacity of the adsorbent and thus the overall adsorptive separation process may decrease as such compounds accumulate on the adsorbent. Methods employed to prevent poisons from deactivating the molecular sieves used to separate olefins are described in US 5276246 A, US 5300715 A and US 6106702 A.

Hence, in a preferred embodiment of the process of the present invention, a guard bed, for example a molecular sieve guard bed may be used to remove aromatic hydrocarbon contaminants from the feed stream in order to prevent such contaminants from deactivating the adsorbent bed used to separate olefins from paraffins in the feed stream. The guard bed may be the same or different from the adsorbent bed used to effect separation of olefins from the feed stream. Suitable guard beds include those described in US 6102702 A and US 6225518 B1. A preferred molecular sieve for use in the guard bed is a lithium exchanged X zeolite.

US 5300715 A, US 6106702 A and US 6225518 B1 describe methods to regenerate molecular sieves used as a guard bed and/or as the adsorbent bed in order to remove remaining diolefins (for example, that were not removed in a previous optional selective hydrogenation step) and aromatic hydrocarbons therefrom and allow said molecular sieves to be re-used.

Adsorption and desorption conditions used in the process of the present invention may be varied depending upon on the composition of the adsorbent and the feed stream. However, the adsorptive separation process of the present invention may be preferably carried out at a temperature in the range of from 20 to 250 °C, more preferably in the range of from 40 to 200 °C and most preferably in the range of from 50 to 150 °C. The process of the present invention may be preferably carried out at a pressure sufficient to maintain the process fluids in liquid phase, for example at a pressure in the range of from atmospheric pressure to 4.5 MPa. Desorption conditions are generally similar to adsorption conditions, that is to say at a temperature in the range of from 20 to 250 °C, more preferably in the range of from 40 to 200 °C and most preferably in the range of from 50 to 150 °C and a pressure sufficient to maintain the process fluids in liquid phase, for example at a pressure in the range of from atmospheric pressure to 4.5 MPa.

The process of the present invention is not limited to any one particular flow scheme.

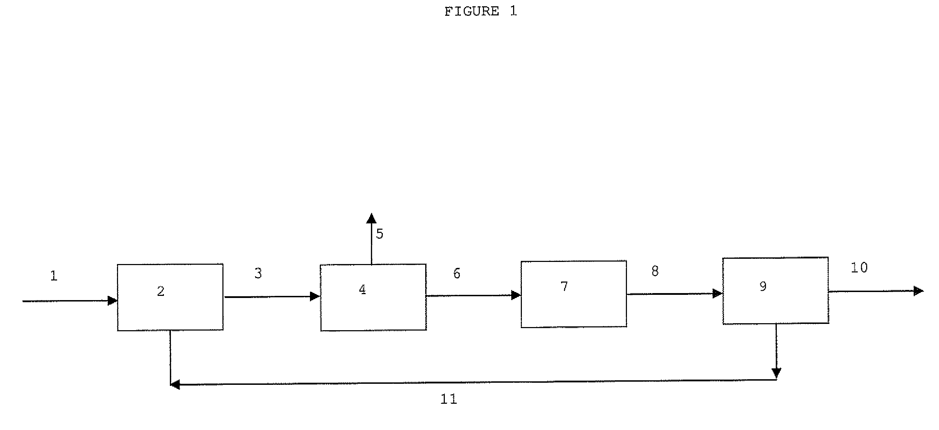

However, in a preferred embodiment of the present invention, the process may be configured substantially in the manner shown in the scheme of Figure 1. Briefly, a feed stream containing Fischer-Tropsch derived paraffins (1) combines with recycled hydrogen to form a dehydrogenation reactant stream that is heated and contacted with a dehydrogenation catalyst in a fixed bed in a dehydrogenation zone (2) maintained at dehydrogenation conditions. The effluent of the fixed catalyst bed, which is referred to herein as the dehydrogenation reactor effluent stream (3), is cooled, partially condensed, and passed to a vapour-liquid separator (4). The vapour-liquid separator produces a hydrogen-rich vapour phase (5) and a hydrocarbon-rich liquid phase (6). Diolefins in the hydrocarbon-rich liquid phase (6) may be optionally hydrogenated to monoolefins. The condensed liquid phase (6) recovered from the separator then passes to a stripping column (7), which removes all compounds which are more volatile than the lightest hydrocarbon which is desired. The monoolefin-containing net stream is referred to herein as the dehydrogenated product stream (8). The dehydrogenated product stream is passed to a separation zone as described herein (9) to separate the monoolefins (the so-called "extract stream") (10) from the unreacted paraffins (11) by an adsorptive separation process, which unreacted paraffins (the so-called "raffinate stream") (11) may, in turn, be optionally recycled to the dehydrogenation zone (2). In a preferred embodiment of the process of the present invention, said dehydrogenated product stream (8) is passed over a guard bed prior to entering the separation zone (9) in order to remove aromatic by-products and any remaining diolefins therefrom.

In a preferred embodiment of the process of the present invention, at least a portion of the separated monoolefins is contacted with a sulphonating agent and the resulting product is subsequently treated to form an olefin sulphonate surfactant, in particular an internal olefin sulphonate surfactant.

The sulphonation may be carried out batchwise, semi-continuously or continuously, preferably continuously.

Sulphonation and subsequent treatment to form an olefin surfactant sulphonate may be conveniently carried out in accordance with the methods described in US 4393937 A, EP 0351928 A1 and EP 0482687 A1.

Examples of sulphonating agents that may be conveniently used include not only sulphur trioxide (SO₃) but also any compounds or complexes which contain or yield SO₃. However, sulphur trioxide per se is particularly preferred for use as a sulphonating agent.

Preferably, the sulphonation reaction takes place between the one or more monoolefins and a sulphonating agent, for example in a film reactor, in a mol ratio of sulphonating agent to monoolefin in the range of from 2.0 to 0.9, more preferably in the range of from 1.3 to 1.0, while cooling the reactor with a cooling means having a temperature preferably not exceeding 35 °C and treating the reaction product to form the olefin sulphonate surfactant.

The reaction between the sulphonating agent and a monoolefin yields an intermediate believed to be in the nature of a sultone. The intermediate is subsequently treated to form an olefin sulphonate surfactant.

In one embodiment of the present invention, said treatment may comprise neutralisation by reaction with a base, preferably an alkali or alkaline earth metal hydroxide, oxide or carbonate, followed by a subsequent after-treatment step, i.e. a so-called hydrolysis step.

The neutralisation/hydrolysis may be preferably carried out continuously and at a temperature in the range of from 20 to 50 °C, more preferably in the range of from 30 to 45 °C. Hydrolysis is preferably carried out continuously at a higher temperature than the neutralisation step, for example, up to and including 190 °C.

Another embodiment of the present invention, provides for the use of monoolefins prepared by the afore-mentioned adsorptive separation process of the present invention in the manufacture of olefin sulphonate surfactants, in particular internal olefins sulphonates.

The present invention further provides for the use of olefin sulphonate surfactants, and in particular internal olefin sulphonates, for enhanced oil recovery, wherein said olefin sulphonate surfactants have been made by the afore-mentioned sulphonation process, and wherein at least a portion of the monoolefins reacted in said sulphonation process are from the afore-mentioned extract stream.

## Claims

1. An adsorptive separation process for the separation of olefins having in the range of from 20 to 28 carbon atoms from a feed stream comprising one or more olefins and paraffins, comprising contacting the feed stream with a bed of adsorbent under conditions which cause the selective retention of the olefins having in the range of from 20 to 28 carbon atoms on the adsorbent and recovering the retained olefins having in the range of from 20 to 28 carbon atoms from the adsorbent by contacting the adsorbent with a desorbent.

2. Process according to Claim 1, wherein said olefins are branched olefins having in the range of from 20 to 28 carbon atoms.

3. Process according to Claim 1 or 2, wherein feed stream is derived from a paraffin dehydrogenation process.

4. Process according to any one of Claims 1 to 3, wherein the feed stream is derived from a Fischer-Tropsch process.

5. Process according to any of Claims 1 to 4, wherein the adsorbent comprises a molecular sieve.

6. Process according to any one of Claims 1 to 5, wherein the desorbent is selected from paraffins, naphthenic hydrocarbons, olefins and aromatic hydrocarbons having in the range of from 5 to 18 carbons.

7. Process according to any one of Claims 1 to 6, wherein the adsorptive separation process is a simulated-moving-bed adsorptive separation process.

8. Process according to any one of Claims 1 to 7, wherein at least a portion of the separated olefins having in the range of from 20 to 28 carbon atoms is contacted with a sulphonating agent and the resulting product is subsequently treated to form one or more olefin sulphonate surfactants.

9. Use of olefins having in the range of from 20 to 28 carbon atoms prepared by the process of any one of Claims 1 to 7 in the manufacture of olefin sulphonate surfactants.

10. Use of olefin sulphonate surfactants made by the process of Claim 8 for enhanced oil recovery.
